# FASCICULE DE BREVET EUROPEEN

(11) **EP 1 035 858 B1**
(45) Date de publication et mention de la délivrance du brevet: **24.04.2002**
(21) Numéro de dépôt: 98958285.3
(22) Date de dépôt: 01.12.1998
(51) Int. Cl.: A61K 35/78

(54) **UTILISATION D'EXTRAITS DE GINKGO BILOBA POUR PREPARER UN MEDICAMENT**
VERWENDUNG VON GINKGO BILOBA EXTRAKTEN ZUR HERSTELLUNG EINES MEDIKAMENTS
USE OF GINKGO BILOBA EXTRACTS FOR PREPARING A MEDICINE

(30) Priorité: 03.12.1997 FR 9715230
(43) Date de publication de la demande: 20.09.2000
(73) Titulaire: SOCIETE DE CONSEILS DE RECHERCHES ET D'APPLICATIONS SCIENTIFIQUES (S.C.R.A.S.), 75016 Paris (FR)
(72) Inventeur: DRIEU, Katy, F-75015 Paris (FR)
(74) Mandataire: Bourgouin, André
(86) Numéro de dépôt international: FR9802576
(87) Numéro de publication internationale: WO9927943

(56) Documents cités:
- EP-A- 0 431 535
- EP-A- 0 431 536
- EP-A- 0 436 129
- PATENT ABSTRACTS OF JAPAN vol. 018, no. 046 (C-1157), 25 janvier 1994 & JP 05 271083 A (DAICEL CHEM IND LTD;OTHERS: 01), 19 octobre 1993

## Description

L'invention concerne l'utilisation d'extraits de Ginkgo biloba pour préparer un médicament destiné à faciliter le sevrage d'individus dépendants de la consommation d'une substance engendrant une dépendance et/ou une addiction, telle notamment l'alcool, les amphétamines, le tabac, les drogues toxicomanogènes.

Il est déjà connu que les extraits de Ginkgo biloba présentent une activité dans le domaine cardio-vasculaire (notamment la réduction de l'adhésion des plaquettes), dans le domaine nerveux central (notamment une activité neuroprotective) ou dans le système neuro-sensoriel (notamment une protection rétinienne) ; cf. par exemple DeFeudis et coll., Ginkgo Biloba Extract (EGb 761® ), Pharmaceutical Activities and Clinical applications (Elsevier, Paris, 1991). Leur préparation a fait l'objet d'un certain nombre de brevets, parmi lesquels on peut citer les brevets européens EP 431 535 et EP 431 536, et le brevet américain US 5,389,370.

Or la demanderesse vient de trouver que certains extraits de Ginkgo biloba possèdent en outre de nouvelles propriétés pharmacologiques intéressantes, à savoir de faciliter le sevrage de sujets alcooliques ou drogués, et de façon plus générale de sujets dépendants d'une substance engendrant une dépendance et/ou une addiction. Ella a pu constater que l'administration de ces extraits a pour effet une atténuation des symptômes de sevrage.

L'invention a donc pour objet l'utilisation de ces extraits pour préparer un médicament destiné à faciliter le sevrage d'individus dépendants de la consommation d'une substance engendrant une dépendance et/ou une addiction, telle notamment l'alcool, les amphétamines, le tabac, les drogues toxicomanogènes.

Par drogues toxicomanogènes, on entend notamment la morphine et ses dérivés, l'opium et les drogues opiacées, la cocaïne, le crack, et de façon plus générale toutes les substances, y compris toute substance médicamenteuse, dont un sujet peut devenir dépendant

Par extrait de Ginkgo biloba, on entend au moins un des composés individuels qui puisse être obtenu par extraction de l'arbre de *Ginkgo biloba L.,* et notamment un composé flavonoïdique ou un terpène tel un ginkgolide ou un bilobalide, ou encore un mélange de ces derniers. De préférence, l'extrait utilisé sera tel qu'il contienne une quantité efficace de ginkgolides. Pour les utilisations selon l'invention, on pourra par exemple choisir un extrait de type EGb 761 ou CP 401.

Par ginkgolide, on entend tous les ginkgolides naturels obtenus à partir de l'arbre de Ginkgo biloba, ainsi que les ginkgolides synthétiques et leurs dérivés (résultant par exemple d'une réaction d'acétylation ou d'alkoxylation) et sels pharmaceutiquement actifs. Les ginkgolides utilisés pourront par exemple être le ginkgolide A, le ginkgolide B, le ginkgolide C, le ginkgolide J ou le ginkgolide M (structures données dans le schéma ci-dessous ; ces composés peuvent être isolés à partir d'extraits de feuilles de *Ginkgo biloba -* voir *GINKGOLIDES, Chemistry, Biology, Pharmacology and Clinical Perspectives,* Edité par P. Braquet, J.R. Prous Science Publishers, notamment Volumes 1 (1988) et 2 (1989)). On pourra également utiliser des dérivés glycosylés de ginkgolides ou de dérivés alkoxylés ou acétylés de ginkgolides. Par dérivé alkoxylé de ginkgolide, on entend un dérivé de ginkgolide comportant au moins un groupe alkoxy, linéaire ou ramifié, à la place d'un groupe hydroxy (ces composés sont décrits dans la demande de brevet français FR 88.14392). De la même façon, par dérivé acétylé de ginkgolide, on entend un dérivé de ginkgolide comportant au moins un groupe acétate à la place d'un groupe hydroxy.

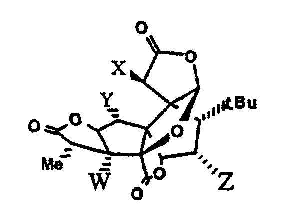

| *Structure des ginkgolides A, B C, J et M* | | | | |
|---|---|---|---|---|
| Ginkgolide | W | X | Y | Z |
| A | OH | OH | H | H |
| B | OH | OH | OH | H |
| C | OH | OH | OH | OH |
| J | OH | OH | H | OH |
| M | H | OH | OH | OH |

Par extrait de type EGb 761, on entend un extrait de composition sensiblement identique à celle de l'extrait standardisé EGb 761 tel qu'il a été défini notamment dans l'article suivant : K. Drieu, La presse médicale, **31**, 25 septembre 1986, supplément consacré à l'extrait de Ginkgo biloba (EGb 761), 1455-1457 ; ou dans les brevets européens EP 431 535 et EP 431 536 ; par extrait de type EGb 761, on entend donc notamment des extraits de Ginkgo biloba comprenant de 20 à 30 % de flavoneglycosides, de 2,5 à 4,5 % de ginkgolides A, B, C et J, de 2 à 4 % de bilobalide, moins de 10 % de proanthocyanidines et moins de 10 ppm, et de préférence moins de 5 ppm, de composés de type alkylphénols, et en particulier les extraits de Ginkgo biloba comprenant environ 24 % de flavoneglycosides, 3,1 % de ginkgolides A, B, C et J, 2,9 % de bilobalide, 6,5 % de proanthocyanidines et moins de 1 ppm de composés de type alkylphénols. Par extrait de type CP 401, on entend des extraits tels que ceux qui sont présentés dans le brevet US 5,389,370, notamment les extraits de Ginkgo biloba contenant de 5,5 à 8 % de ginkgolides A, B, C et J, de 40 à 60 % de flavoneglycosides et de 5 à 7 % de bilobalide, et tout particulièrement les extraits contenant environ 7 % de ginkgolides A, B, C et J, 50 % de flavoneglycosides et 6 % de bilobalide.

Selon un autre aspect de l'invention, l'extrait de Ginkgo biloba utilisé comportera plus de 5 % de ginkgolides, et plus préférentiellement plus de 50 % de ginkgolides.

L'invention concerne aussi l'utilisation d'un ginkgolide ou d'un de ses dérivés ou sels pharmaceutiquement actifs pour préparer un médicament destiné à faciliter le sevrage d'individus dépendants de la consommation d'une substance engendrant une dépendance et/ou une addiction, telle notamment l'alcool, les amphétamines, le tabac, les drogues toxicomanogènes. De préférence, le ginkgolide utilisé pour cet aspect de l'invention sera le ginkgolide A ou le ginkgolide B.

L'invention concerne également l'utilisation d'un composé de formule générale (I) 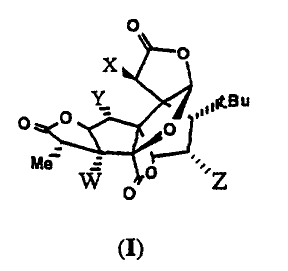 dans laquelle W, X, Y et Z représentent indépendamment les radicaux H, OH, alkoxy linéaire ou ramifié ou O-Gₛ, Gₛ-OH représentant un mono- ou un disaccharide, ou un de leurs dérivés ou analogues,
étant entendu que l'un au moins de W, X, Y ou Z représente un radical O-Gₛ,
pour préparer un médicament destiné à faciliter le sevrage d'individus dépendants de la consommation d'une substance engendrant une dépendance et/ou une addiction, telle notamment l'alcool, le tabac, les amphétamines, les drogues toxicomanogènes.

L'invention concerne de préférence l'utilisation d'un composé de formule générale (I) 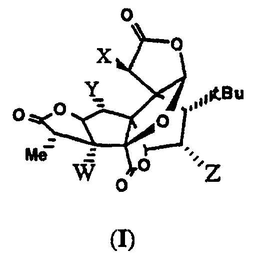 dans laquelle X représente un radical OH ou O-Gₛ, Gₛ-OH représentant un mono- ou un disaccharide, ou un de leurs dérivés ou analogues, et :
- ou bien W représente un radical OH ou O-Gₛ, Y représente H et Z représente H ;
- ou bien W représente un radical OH ou O-Gₛ, Y représente un radical OH ou O-Gₛ et Z représente H ;
- ou bien W représente un radical OH ou O-Gₛ, Y représente un radical OH ou O-Gₛ et Z représente un radical OH ou O-Gₛ ;
- ou bien W représente un radical OH ou O-Gₛ, Y représente H et Z représente un radical OH ou O-Gₛ ;
- ou bien W représente H, Y représente un radical OH ou O-Gₛ et Z représente un radical OH ou O-Gₛ ;
- ou bien W représente un radical OH ou O-Gₛ, Y représente un radical alkoxy linéaire ou ramifié et Z représente H ;
étant entendu que l'un au moins de W, X, Y ou Z représente un radical O-Gₛ,
pour préparer un médicament destiné à faciliter le sevrage d'individus dépendants de la consommation d'une substance engendrant une dépendance et/ou une addiction, telle notamment l'alcool, le tabac, les amphétamines, les drogues toxicomanogènes.

L'invention concerne tout particulièrement l'utilisation d'un composé de formule générale (**I**) 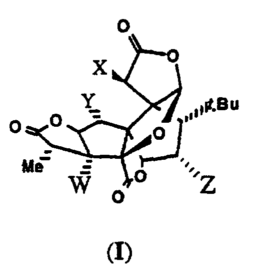 dans laquelle X représente un radical OH ou O-Gₛ, Gₛ-OH représentant un mono- ou un disaccharide, ou un de leurs dérivés ou analogues, et :
- ou bien W représente un radical OH ou O-Gₛ, Y représente H et Z représente H ;
- ou bien W représente un radical OH ou O-Gₛ, Y représente un radical OH ou O-Gₛ et Z représente H ;
- ou bien W représente un radical OH ou O-Gₛ, Y représente un radical alkoxy linéaire ou ramifié et Z représente H ;
étant entendu que l'un au moins de W, X, Y ou Z représente un radical O-Gₛ,
pour préparer un médicament destiné à faciliter le sevrage d'individus dépendants de la consommation d'une substance engendrant une dépendance et/ou une addiction, telle notamment l'alcool, le tabac, les amphétamines, les drogues toxicomanogènes.

Par radical alkoxy linéaire ou ramifié, on entend dans la présente description un radical alkoxy dont la chaîne carbonée, linéaire ou ramifiée, compte de 1 à 6 atomes de carbone. Par dérivé ou analogue des mono- ou disaccharides, on entend des composés comme la N-acétylglucosamine, la N-acétylalosamine, la galactosamine, la mannoseamine, la N-tosylhydrazone, etc.

De préférence, O-Gₛ sera choisi de telle sorte que Gₛ-OH appartienne au groupe composé de l'abéquose, du rhamnose, de l'arabinose, du ribose, du xylose, du 2-déoxy-ribose, du glucose, du galactose, du mannose, du 2-déoxyglucose, du fructose, du fucose, de la N-acétylglucosamine, de la N-acétylalosamine, de la galactosamine, de la mannosamine, du saccharose, du lactose, du maltose, du cellobiose et du tréhalose. De façon encore plus préférentielle, O-Gₛ sera choisi de telle sorte que Gₛ-OH appartienne au groupe composé du glucose et du lactose.

L'invention concerne donc également l'utilisation de dérivés glycosylés des ginkgolides, plus particulièrement ceux des ginkgolides A et B, les groupes glycosyle adaptés pour l'invention étant décrits précédemment.

Les différents procédés pour obtenir les dérivés glycosylés des ginkgolides ou des ginkgolides alkoxylés (c'est à dire ceux résultant d'une réaction de glycosylation effectuée sur au moins un des groupes OH des ginkgolides ou de leurs dérivés alkoxylés) sont décrits dans la publication suivante : Weber, M. et Vasella, A., *Helv. Chim. Acta,* **80** (1997), 2352-2367.

Les compositions pharmaceutiques comprenant un composé de l'invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse,etc.), etc.

La dose d'administration envisagée pour un médicament selon l'invention est comprise entre 0,1 mg à 10 g suivant le type de substance dont le sujet à traiter est dépendant.

A moins qu'ils ne soient définis d'une autre manière, tous les termes techniques et scientifiques utilisés ici ont la même signification que celle couramment comprise par un spécialiste ordinaire du domaine auquel appartient cette invention.

### Etude pharmacologique des produits de l'invention :

### 1. Etude des effets des extraits de Ginkgo biloba sur la dépendance de l'alcool :

Deux études ont été menées : l'une porte sur les effets de l'EGb 761, l'autre sur les effets d'un autre extrait de Ginkgo biloba, le CP 401, lequel ne contient pas de bilobalide mais deux fois plus de ginkgolides que l'EGb 761 (6 %).

**1)** Des rats sont traités pendant 15 jours avec de l'alcool (on leur donne de l'éthanol à 10 % dans leur eau de boisson pendant la première semaine et de l'éthanol à 12,5 % ensuite). On leur administre par voie orale (gavage) 50 ou 100 mg/kg d'EGb 761 par jour durant les 5 jours qui précèdent l'arrêt de l'absorption d'alcool (à partir du 11^{e} jour) et les 3 jours qui suivent cet arrêt.

Les symptomes comportementaux ont été évalués durant les 3 jours suivant l'arrêt de l'absorption d'alcool chez trois groupes de rats (n=6) : le groupe témoin n'ayant reçu que de l'alcool, un groupe ayant reçu l'alcool et un traitement à 50 mg/kg d'EGb 761 et un autre groupe ayant reçu l'alcool et un traitement à 100 mg/kg d'EGb 761, les traitements à l'EGb 761 ayant été administrés dans les conditions décrites ci-dessus.

On constate chez les animaux ayant reçu de l'EGb 761 que les symptômes de sevrage (7 critères) sont diminués de façon dose-dépendante et que les animaux ont également une hyperactivité motrice diminuée.

**2)** Des rats sont traités pendant 15 jours avec de l'alcool (on leur donne de l'éthanol à 10 % dans leur eau potable pendant la première semaine et de l'éthanol à 12,5 % ensuite). On leur administre par voie orale (gavage) 50 mg/kg d'extrait CP 401 par jour durant les 5 jours qui précèdent l'arrêt de l'absorption d'alcool (à partir du 11^{e} jour) et les 3 jours qui suivent cet arrêt.

Les symptomes comportementaux ont été évalués durant les 3 jours suivant l'arrêt de l'absorption d'alcool chez trois groupes de rats (n=6) : le groupe témoin n'ayant reçu que de l'alcool et l'autre groupe ayant reçu l'alcool et un traitement à 50 mg/kg d'extrait CP 401 administré dans les conditions décrites ci-dessus.

On constate que les animaux qui ont reçu l'extrait CP 401 présentent une diminution des symptômes liés au sevrage par rapport aux animaux témoins intoxiqués.

### 2. Etude des effets des extraits de Ginkgo biloba sur la sensibilisation à l'amphétamine :

Une injection d'amphétamine (0,5 mg/kg IP) entraîne chez le rat une hyperactivité motrice (mesurée par actimétrie). L'administration, répétée tous les deux jours, huit fois, de la même dose d'amphétamine entraîne une augmentation progressive de l'activité locomotrice : ce phénomène est appelé "sensibilisation".

Des rats (n=8) soumis à l'administration d'amphétamine telle que décrite ci-dessus ont été soumis les 8 jours qui précèdent l'administration d'amphétamine et pendant toute la durée cette administration à un traitement par voie orale d'une dose d'EGb 761 de 100 mg/kg par jour ou d'une dose de 5 mg/kg par jour de ginkgolide A.

La mesure d'actimétrie a été effectuée durant 1 h après l'administration de l'amphétamine aux 9^{e} (premier jour d'administration d'amphétamine), 13^{e}, 17^{e}, 21^{e} et 25^{e} jour Les résultats de ces essais sont représentés sur la A que l'on trouvera en annexe I.

On constate que la sensibilisation comportementale à l'amphétamine est réduite chez les animaux qui ont reçu du ginkgolide A à 5 mg/kg par jour. Un effet encore meilleur et tout à fait significatif est observé avec EGb 761 à 100 mg/kg par jour .

### 3. Etude des effets de l'extrait de Ginkgo biloba EGb 761 sur le syndrome de sevrage morphinique :

Des rats sont traités toutes les 8 heures (3 fois par jour) pendant 10 jours avec une dose de morphine par voie sous-cutanée entraînant une hyperactivité motrice (mesure par actimétrie). Le 11^{e} jour, on leur administre de la naloxone (3 mg/kg IP) et les signes de sevrage sont observés pendant 60 minutes : une série de signes comportementaux est comptabilisée, une série mesurée (hypothermie, perte de poids) ou bien une série côtée (échelle à 4 niveaux).

Deux groupes de 8 rats sont traités par l'EGb 761 (50 ou 100 mg/kg par jour) pendant 4 jours avant l'administration de naloxone et 2 heures avant celle-ci. Un groupe de rats témoins intoxiqués ne reçoit que les injections de morphine avant la naloxone et un groupe témoins absolus ne reçoit que la naloxone.

L'analyse statistique entre les lots est faite avec les tests suivants : Anova paramétrique, test de Barlett pour vérifier l'homogénéité des variances et test de Dunnett pour comparaisons multiples.

Les résultats quantifiés par comptage pour les différents paramètres comportementaux analysés sont repris dans le tableau que l'on trouvera en annexe II.

### ANNEXE I

### Effet des substances EGb 761 et Ginkgolide A sur la sensibilisation à l'amphétamine

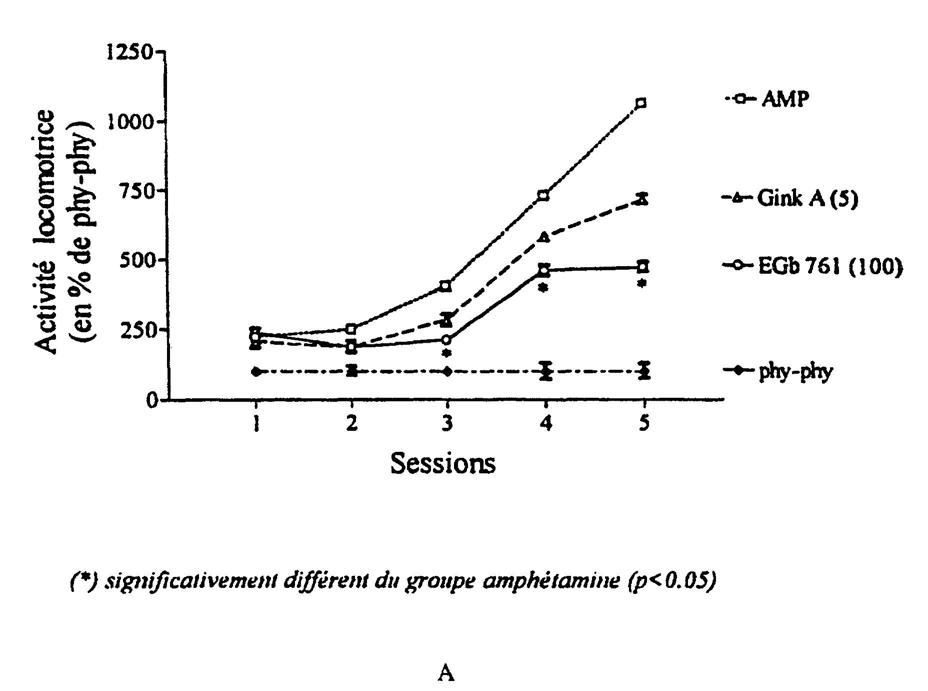

### ANNEXE II

**Tableau II**

| *Influence d'un traitement par la substance EGb 761 sur le nombre d'observations de chacun des symptômes d'abstinence lors d'un sevrage morphinique* | | | |
|---|---|---|---|
| **Symptômes** | **Groupe 1** | **Groupe 2** | **Groupe 3** |
| **Sauts** | 0,0 ± 0,0 | 1,00 ± 0,33 | 0,50 ± 0,19 |
| **Redressements** | 9,88 ± 1,03 | 1,13 ± 0,40 | 5,63 ± 1,00 |
| **Ebrouements** | 0,25 ± 0,16 | 2,75 ± 0,70 | 0,88 ± 0,29 |
| **Saccades de la tête** | 0,0 ± 0,0 | 5,50 ± 1,13 | 2,43 ± 0,48 |
| **Baillements** | 0,75 ± 0,41 | 2,00 ± 0,78 | 0,88 ± 0,40 |
| **Claquements ou grincements de dents** | 0,0 ± 0,0 | 4,75 ± 0,86 | 1,75 ± 0,45 |
| **Enfouissements** | 0,25 ± 0,16 | 1,38 ± 0,46 | 0,25 ± 0,16 |
| **Grattages excessifs** | 0,0 ± 0,0 | 1.13 ± 0,48 | 0,38 ± 0,26 |
| **Toilettes** | 6,00 ± 1,39 | 1,38 ± 0.53 | 4,25 ± 1.31 |

### Légende:

Groupe 1 : contrôle ;
Groupe 2 : groupe traité uniquement à la morphine (3 fois 10 mg/kg/jour) ;
Groupe 3 : groupe traité à la morphine et à l'EGb 761 à une dose de 100 mg/kg.

## Revendications

1. Utilisation d'un extrait de Ginkgo biloba pour préparer un médicament destiné à faciliter le sevrage d'individus dépendants de la consommation d'une substance engendrant une dépendance et/ou une addiction, telle notamment l'alcool, les amphétamines, le tabac, les drogues toxicomanogènes.

2. Utilisation selon la revendication 1, **caractérisé en ce que** l'extrait de Ginkgo biloba est un extrait de type EGb 761.

3. Utilisation selon la revendication 1, **caractérisé en ce que** l'extrait de Ginkgo biloba est un extrait de type CP 401.

4. Utilisation selon la revendication 1, **caractérisée en ce que** l'extrait de Ginkgo biloba contient au moins 5 % de ginkgolides.

5. Utilisation selon la revendication 4, **caractérisée en ce que** l'extrait de Ginkgo biloba contient au moins 50 % de ginkgolides.

6. Utilisation d'un composé de formule générale **(I)** 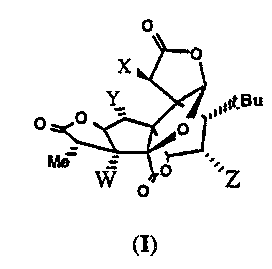 dans laquelle W, X, Y et Z représentent indépendamment les radicaux H, OH, alkoxy linéaire ou ramifié ou O-G_{S}, Gₛ-OH représentant un mono- ou un disaccharide, ou un de leurs dérivés ou analogues,
étant entendu que l'un au moins de W, X, Y ou Z représente un radical O-G_{S},
pour préparer un médicament destiné à faciliter le sevrage d'individus dépendants de la consommation d'une substance engendrant une dépendance et/ou une addiction, telle notamment l'alcool, les amphétamines, le tabac, les drogues toxicomanogènes.

7. Utilisation selon la revendication 6, **caractérisée en ce que**:
- ou bien W représente un radical OH ou O-Gₛ, Y représente H et Z représente H ;
- ou bien W représente un radical OH ou O-Gₛ, Y représente un radical OH ou O-Gₛ et Z représente H ;
- ou bien W représente un radical OH ou O-Gₛ, Y représente un radical OH ou O-Gₛ et Z représente un radical OH ou O-Gₛ ;
- ou bien W représente un radical OH ou O-Gₛ, Y représente H et Z représente un radical OH ou O-Gₛ ;
- ou bien W représente H, Y représente un radical OH ou O-Gₛ et Z représente un radical OH ou O-Gₛ ;
- ou bien W représente un radical OH ou O-Gₛ, Y représente un radical alkoxy linéaire ou ramifié et Z représente H ;
étant entendu que l'un au moins de W, X, Y ou Z représente un radical O-Gₛ.

8. Utilisation selon la revendication 6 ou 7, **caractérisée en ce que** :
- ou bien W représente un radical OH ou O-Gₛ, Y représente H et Z représente H ;
- ou bien W représente un radical OH ou O-Gₛ, Y représente un radical OH ou O-Gₛ et Z représente H ;
- ou bien W représente un radical OH ou O-Gₛ, Y représente un radical alkoxy linéaire ou ramifié et Z représente H ;
étant entendu que l'un au moins de W, X, Y ou Z représente un radical O-Gₛ.

9. Utilisation d'un ginkgolide ou d'un de ses dérivés glycosylés, alkoxylés ou acétylés, ou d'un sel pharmaceutiquement actif de ces derniers pour préparer un médicament destiné à faciliter le sevrage d'individus dépendants de la consommation d'une substance engendrant une accoutumance et/ou une assuétude, telle notamment l'alcool, les amphétamines, le tabac, les drogues toxicomanogènes.

10. Utilisation selon la revendication 9, **caractérisée en ce que** le ginkgolide est le ginkgolide A ou le ginkgolide B.

## Claims

1. Use of a Ginkgo biloba extract for preparing a medicament intended to ease the withdrawal of individuals dependent on the consumption of a substance engendering dependency and/or addiction, such as in particular alcohol, amphetamines, tobacco, drugs inducing toxicomania.

2. Use according to claim 1, **characterized in that** the Ginkgo biloba extract is an extract of type EGb 761.

3. Use according to claim 1, **characterized in that** the Ginkgo biloba extract is an extract of type CP 401.

4. Use according to claim 1, **characterized in that** the Ginkgo biloba extract contains at least 5 % ginkgolides.

5. Use according to claim 4, **characterized in that** the Ginkgo biloba extract contains at least 50 % ginkgolides.

6. Use of a compound of general formula **(I)** 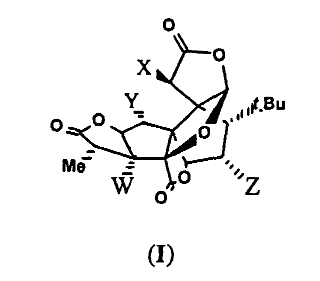 in which W, X, Y and Z independently represent the H, OH, linear or branched alkoxy or O-Gₛ radicals, Gₛ-OH representing a mono- or a disaccharide, or one of their derivatives or analogues,
it being understood that at least one of W, X, Y or Z represents an O-Gₛ radical,
for preparing a medicament intended to ease the withdrawal of individuals dependent on the consumption of a substance engendering dependency and/or addiction, such as in particular alcohol, amphetamines, tobacco, drugs inducing toxicomania.

7. Use according to claim 6, **characterized in that**:
- either W represents an OH or O-Gₛ radical, Y represents H and Z represents H;
- or W represents an OH or O-Gₛ radical, Y represents an OH or O-Gₛ radical and Z represents H;
- or W represents an OH or O-Gₛ radical, Y represents an OH or O-Gₛ radical and Z represents an OH or O-Gₛ radical;
- or W represents an OH or O-Gₛ radical, Y represents H and Z represents an OH or O-G_{S} radical;
- or W represents H, Y represents an OH or O-Gₛ radical and Z represents an OH or O-Gs radical;
- or W represents an OH or O-Gₛ radical, Y represents a linear or branched alkoxy radical and Z represents H;
it being understood that at least one of W, X, Y or Z represents an O-Gₛ. radical

8. Use according to claim 6 or 7, **characterized in that**:
- either W represents an OH or O-Gₛ radical, Y represents H and Z represents H;
- or W represents an OH or O-Gₛ radical, Y represents an OH or O-Gₛ radical and Z represents H;
- or W represents an OH or O-Gₛ radical, Y represents a linear or branched alkoxy radical and Z represents H;
it being understood that at least one of W, X, Y or Z represents an O-Gₛ. radical

9. Use of a ginkgolide or one of its glycosylated, alkoxylated or acetylated derivatives or a pharmaceutically active salt for preparing a medicament intended to ease the withdrawal of individuals dependent on the consumption of a substance engendering habituation and/or addiction, such as in particular alcohol, amphetamines, tobacco, drugs inducing toxicomania.

10. Use according to claim 9, **characterized in that** the ginkgolide is ginkgolide A or ginkgolide B.

## Patentansprüche

1. Verwendung eines Ginkgo-biloba-Extrakts für die Herstellung eines Medikaments, das zur Unterstützung der Entwöhnung von Patienten bestimmt ist, die vom Konsum einer eine Abhängigkeit und/oder eine Sucht erzeugenden Substanz abhängig sind, wie insbesondere Alkohol, Amphetamine, Tabak, toxikomanogene Drogen.

2. Verwendung nach Anspruch 1, **dadurch gekennzeichnet, dass** der Ginkgo-biloba-Extrakt ein Extrakt vom Typ EGb 761 ist.

3. Verwendung nach Anspruch 1, **dadurch gekennzeichnet, dass** der Ginkgo-biloba-Extrakt ein Extrakt vom Typ CP 401 ist.

4. Verwendung nach Anspruch 1, **dadurch gekennzeichnet, dass** der Ginkgo-biloba-Extrakt mindestens 5 % Ginkgolide enthält.

5. Verwendung nach Anspruch 4, **dadurch gekennzeichnet, dass** der Ginkgo-biloba-Extrakt mindestens 50 % Ginkgolide enthält.

6. Verwendung einer Verbindung der allgemeinen Formel (I) 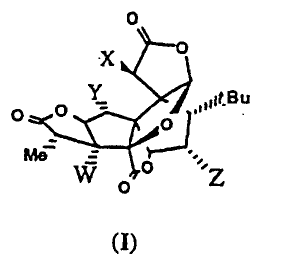 in der W, X, Y und Z unabhängig voneinander die Reste H, OH, verzweigtes oder unverzweigtes Alkoxy oder O-Gₛ, wobei Gₛ-OH ein Mono- oder ein Disaccharid darstellt, oder eines ihrer Derivate oder Analoga darstellen,
wobei von W, X, Y oder Z mindestens eines einen O-Gₛ-Rest darstellt,
für die Herstellung eines Medikaments, das zur Unterstützung der Entwöhnung von Patienten bestimmt ist, die vom Konsum einer eine Abhängigkeit und/oder eine Sucht erzeugenden Substanz abhängig sind, wie insbesondere Alkohol, Amphetamine, Tabak, toxikomanogene Drogen.

7. Verwendung nach Anspruch 6, **dadurch gekennzeichnet, dass**
- W einen OH- oder O-Gₛ-Rest, Y H und Z H darstellen
- oder W einen OH- oder O-Gₛ-Rest, Y einen OH- oder O-Gₛ-Rest und Z H darstellen
- oder W einen OH- oder O-Gₛ-Rest Y einen OH- oder O-Gₛ-Rest und Z einen OH- oder O-Gₛ-Rest darstellen
- oder W einen OH- oder O-Gₛ-Rest, Y H und Z einen OH- oder O-Gₛ-Rest darstellen
- oder W H, Y einen OH- oder O-Gₛ-Rest und Z einen OH- oder O-Gₛ-Rest darstellen
- oder W einen OH- oder O-Gₛ-Rest, Y einen verzweigten oder unverzweigten Alkoxyrest und Z H darstellen,
wobei von W, X, Y oder Z mindestens eines einen O-Gₛ-Rest darstellt.

8. Verwendung nach Anspruch 6 oder 7, **dadurch gekennzeichnet, dass**
- W einen OH- oder O-Gₛ-Rest, Y H und Z H darstellen
- oder W einen OH- oder O-Gₛ-Rest, Y einen OH- oder O-Gₛ-Rest und Z H darstellen
- oder W einen OH- oder O-Gₛ-Rest, Y einen verzweigten oder unverzweigten Alkoxyrest und Z H darstellen,
wobei von W, X, Y oder Z mindestens eines einen O-Gₛ-Rest darstellt.

9. Verwendung eines Ginkgolids oder eines seiner glykosylierten, alkoxylierten oder acetylierten Derivate oder eines pharmazeutisch aktiven Salzes von diesen für die Herstellung eines Medikaments, das zur Unterstützung der Entwöhnung von Patienten bestimmt ist, die vom Konsum einer eine Gewöhnung und/oder eine Sucht erzeugenden Substanz abhängig sind, wie insbesondere Alkohol, Amphetamine, Tabak, toxikomanogene Drogen.

10. Verwendung nach Anspruch 9, **dadurch gekennzeichnet, dass** das Ginkgolid das Ginkgolid A oder das Ginkgolid B ist.
